# EUROPEAN PATENT APPLICATION

(11) **EP 2 248 908 A1**
(43) Date of publication of application: **10.11.2010**
(21) Application number: 09159379.8
(22) Date of filing: 05.05.2009
(51) Int. Cl.: C12P 21/02, C12N 1/20, A23C 9/12, A61K 35/74, C12R 1/25, C12R 1/225

(54) **Probiotic microorganisms isolated from donkey milk**

(71) Applicant: Eurolactis Group S.A., 1420 Luxembourg (LU)
(72) Inventor: Nazzaro, Filomena, 80055 Portici (NA) (IT); Orlando, Pierangelo, 80078 Pozzuoli (NA) (IT); Conti, Amedeo, 14022 Castelnuovo don Bosco (AT) (IT)
(74) Representative: GLN

(57) **Abstract**

Probiotic lactic bacteria of the Lactobacillus genus, isolated from Donkey milk, are disclosed, as well as their use in food or beverage compositions such as fermented milk products.

## Description

### Technical Field

The invention relates to the field of micro-organisms known as probiotics and their use in the food industries, in human or animal food. In particular, the invention relates to probiotics isolated from raw Donkey milk.

### Background of the invention

The probiotic micro-organisms, or simply probiotics can be defined as micro-organisms which, when administered in adequate doses, are likely to confer a benefit in terms of health or nutrition. They belong to the category of so-called functional foods.

Probiotics are known in dairy products, such as products marketed under the Actimel® or Activia® brands by DANONE, Yakult® brand by Yakult Honsha, or under the LC1® brand by NESTLE. These bacteria belong to different genera and species of lactic acid bacteria, for example Bifidus spp. Lactobacillus casei, Lactobacillus rhamnosus or Lactobacillus johnsonii.

It is generally accepted that probiotics provide a health or nutritional benefit through the influence they can have on the balance of the intestinal flora, though the precise mechanisms by which probiotics act are not always known.

In recent years, the interest in Donkey milk has been considerably increased, mainly due to its composition, so that it may be considered a valid alternative for infant nutrition to powdered milks, soybean milk or other formulas. In fact Donkey milk is viewed as very close to the human milk due to its composition in poly-unsatured fatty acids, its calcium/phosphorous ratio and its protein content. In addition, Donkey milk is rich of lysozyme, a glycosidase capable to hydrolyze the polysaccharides of the microbial cell wall. Several scientific evidence proves the nutritional and health importance of lysozyme. The high content of lactose has a positive effect on the intestinal absorption of calcium and it is responsible for palatability. Even after the first months of life, Donkey milk can enhance bone mineralization and constitutes an important nutritional support in those children with severe Ig-E mediated cow milk protein allergy, thus playing a role in the formation of an efficient immune system. In the elderly age, Donkey milk can exert positive effects against different cardiovascular pathologies and in hypocholesterolemic diets.

### Summary of the invention

It is an object of the invention to provide novel probiotic lactic bacteria.

To this end, an embodiment of the invention proposes probiotic lactic bacteria isolated from Donkey milk. In an embodiment, said bacteria may be selected among the species Lactobacillus plantarum, Lactobacillus paraplantarum, Lactobacillus pentosus, and Lactobacillus asini and among the subspecies Lactobacillus plantarum asini. In a preferred embodiment, the probiotic lactic bacteria is selected from the group consisting of strains deposited under the terms of the Budapest Treaty at the "Deutsche Sammlung von Mikroorganismen und Zellkulturen GmbH" (DSMZ) on December 8, 2008 under accession number DSM 22098, DSM 22099, DSM 22100, DSM 22102 and DSM 22101.

Another embodiment of the invention proposes a composition comprising one or a plurality of the probiotic lactic bacteria species or strains as mentioned above. Said composition could be a food composition or a beverage composition, for human or animal feed.

A further embodiment of the invention provides a process for manufacturing a food composition or a beverage composition. Said process may comprise at least the steps of:
- inoculating a food product with viable probiotic lactic bacteria as described above,
- placing the inoculated food product under conditions favorable to the metabolism of said probiotic lactic bacteria
- fermenting said food product until a population of at least [10⁶] CFU / mL of food product is reached.

In another embodiment, the process for manufacturing a food composition or a beverage composition contains at least the steps of:
- fermenting probiotic lactic bacteria as described above until a population of at least [10⁶] CFU / mL is reached,
- protecting said probiotic lactic bacteria, for example by enveloping,
- admixing a food product with said protected probiotic lactic bacteria.

The fermentation is stopped when it has reached the stationary phase of fermentation, preferably within about 24 hours of having placed said probiotic lactic bacteria under conditions favorable to their metabolism.

Another embodiment of the invention proposes the use of probiotic lactic bacteria in the preparation of a composition for treating a disorder associated with the colonization of the mucuous membranes by pathogenic microorganisms. Mucuous membranes include, but are not limited to the gut mucosa, the stomach mucosa. For instance, pathogenic microorganisms may be enteropathogens. For instance, inhibitory activity of Lactobacillus strains according to the invention, could be against: enteropathogens (for example Salmonella enteriditis, Vibrio cholereae, Escherichia coli).

Another embodiment of the invention proposes the use of probiotic lactic bacteria for protecting fermented food products against food pathogens, such as Lysteria or Salmonella, Campylobacter or Clostridium, by inhibiting the development of such food pathogens.

These and other aspects, features and advantages of the invention will become apparent to those skilled in the art upon reading the disclosure provided here in connection with the attached drawings. The detailed description, while indicating preferred embodiments of the invention, is only given by way of illustration.

### Brief description of the drawings

Figure 1 shows a DNA-fingerprinting of 8 clones isolated from raw Donkey milk.

Figures 2a to 2e show the electrophorograms of clones 37, 38, 41, 43 and 48, respectively, after DNA-fingerprinting analysis.

Figure 3 shows the result of DNA-DNA hybridization between clones 37, 38 and 48, using L. plantarum DNA labelled by dUTP-digoxigenin as a probe.

Figure 4 shows the result of DNA-DNA hybridization between clones 37, 38 and 48 were DNA-DNA hybridised within L. plantarum, L. paraplantarum and L. pentosus strain-type DNAs, to a probe of labelled DNA from clone 37.

### Detailed description

During the past decades, the role of probiotics on the human health gained great relevance both at scientific and industrial level, by causing a sensible increase in their market request, as well as to a greater production and consumption of products. For the viability aspect it is of prime importance that the probiotic strains have the ability to overcome the extremely low pH of gastric acid and the detergent effect of bile salts and to arrive in a viable physiological state at the site of action: the intestinal epithelium. Probiotic bacteria are capable to colonize the colon, to positively affect the infections outcome by pathogenic bacteria, to stimulate the immune system and to decrease unfavourable metabolites concentration. In addition, some strains, the bacteria of the invention being included, can provide a certain reduction of cholesterol and triacylglycerol plasma concentrations.

During passage in the gastro-intestinal (GI) tract, probiotic cultures are required to tolerate the presence of pepsin and the low pH of the stomach, the protease-rich conditions of the duodenum, and the antimicrobial activity of bile salts. Although the pH of the stomach may increase up to 6.0 or higher after food intake, it generally ranges from 2.5 to 3.5. Following stomach passage, the small intestine is a second major barrier in the GI tract. Although the pH of the small intestine (i.e., 7.0 to 8.5) is more favourable toward bacterial survival, the presence of bile salts may have adverse effects. Traditionally, the ability of a probiotic candidate to survive GI transit is assessed using conventional plating techniques that provide information on the number of viable and reproductive cells during incubation in simulated GI juices as described for instance in an article by Charteris et al. « Development of an in vitro methodology to determine the transit tolerance of potentially probiotic Lactobacillus and Bifidobacterium species in the upper gastrointestinal tract » J. Appl. Microbiol. 84 :759-768 (1998).

Therefore, various cultures based methods, such as resistance to gastric juices, resistance to bile salts, bile salt hydrolyzing capability, hydrophobicity evaluation, antimicrobial activity, antibiotic sensitivity, may be used to characterize probiotic micro-organisms. These tests are fully described in the Examples section below.

In an embodiment of the invention, the probiotic bacteria meet at least one of the following criteria :
- at least 75% CFU/mL are retained after the resistance to gastric juice evaluation as described herein,
- at least 75% CFU/mL are retained after the resistance to gastric juice and bile salts evaluation as described herein,
- a microbial aggregation visible onto a microscopy glass with 0.5 M ammonium sulphate or less, preferably with 0.1 M ammonium sulphate or less, in the hydrophobicity test as described herein,
- a low antimicrobial activity against other Lactobacillus spp, as measured by the halos test, i.e. a halo smaller than 0.5 cm, preferably smaller than 0.2 cm as described herein,
- an inhibitory activity against Pseudomonas aeruginosa, Bacillus cereus, or Escherichia coli, as measured by the halo method, i.e. a halo greater than 0.5 cm, preferably greater than 0.8 cm as described herein.

As a complementary, or alternative, definition, the embodiments of the invention also relate to Lactobacillus strains that present a 16 S RNA identity of at least 95% with Clone 37, Clone 38, Clone 48, Clone 43 or Clone 41.

Clones 37, 38, 48, 43 and 41 represent specific embodiments of the invention. They have been deposited on December 8, 2008 with the "Deutsche Sammlung von Mikroorganismen und Zellkulturen GmbH" (DSMZ) in conformity with the Budapest Treaty, under the accession number:

| | |
|---|---|
| Clone 37 | DSM 22098 |
| Clone 38 | DSM 22099 |
| Clone 48 | DSM 22100 |
| Clone 43 | DSM 22102 |
| Clone 41 | DSM 22101. |

Clones 37, 38 and 48 belong to the subspecies Lactobacillus plantarum asini and clones 43 and 41 belong to the species Lactobacillus asini.

Said probiotic bacteria may be provided as a fresh culture or as dried food supplement, such as dietary supplement. In the latter case, the biomass may be freeze-dried or sprayed-dried, to provide a high quality culture powder, comprising for example at least 10⁸CFU/g, preferably over 10⁹ CFU/g.

According to the invention, probiotic bacteria may be used in the preparation of food products or beverages, for human or animal consumption. Food products and beverages include fermented food products, such as fresh dairy products, fermented milks, yoghurts. Milk usually refers to cow milk. In the context of the invention, Donkey milk may be preferred, although other milks may also be used for preparing dairy products, including mare milk, goat milk, camel milk, ewe milk.

Methods for preparing such fermented dairy food products are within the reach of person of ordinary skill in the art. Preferably, the probiotic bacteria a provided or kept in a viable form up until consumption. The milk may be used fresh, or in powder and reconstituted with water. Various ingredients may be added to the milk, to improve fermentation, or to provide additional health benefits to the consumer (such as adding vitamins or minerals).

After fermentation, the milk can be transformed into cottage-cheese or quark, by adding rennet to the fermented milk for instance. Such procedures are well-known to the skilled person.

In another embodiment, the probiotic bacteria are used as an ingredient in a food or beverage composition, without fermentation of said composition. In other words, said probiotic bacteria is a dietary or nutritional supplement. In this case, it is preferred that the probiotic bacteria be provided in a viable form.

Probiotic lactic bacteria according to the invention may also be used to protect food products, such as fermented food products, against food pathogens, such as Lysteria or Salmonella, Campylobacter or Clostridium, by inhibiting the development of such food pathogens. In that case, a composition comprising the desired probiotic bacteria, as disclosed herein, is admixed to a food or beverage product. The latter product may be fermented independently of the composition comprising the desired probiotic bacteria. In any case, it is preferred that fermentation lasts not more than about 24 hours. Usually, the probiotic strains have reached the stationary phase of fermentation within this time period.

In an embodiment of the invention, fermentation is stopped when it has reached the stationary phase of fermentation, preferably within about 24 hours of having placed the inoculated food product under conditions favorable to the metabolism of said probiotic lactic bacteria.

The invention also relates to the use of probiotic lactic bacteria in the preparation of a composition for treating a disorder associated with the colonization of the mucuous membranes by pathogenic microorganisms. Mucuous membranes include, but are not limited to the gut mucosa and the stomach mucosa. For instance, pathogenic microorganisms may be enteropathogens. For instance, inhibitory activity of Lactobacillus strains according to the invention, could be against: enteropathogens (for example Salmonella enteriditis, Vibrio cholereae, Escherichia coli). This is associated with the probiotic bacteria' s capacity to adhere to epithelium cells, such as gut cells and, as it is currently understood, to exclude to a certain degree, such pathogenic bacteria from the gut. In turn, this could be correlated with the hydrophobicity of the probiotic bacteria.

### EXAMPLES

Culture based methods (probiotic tests, bile salt hydrolyzing capability, hydrophobicity evaluation, antimicrobial activity, antibiotic sensitivity)

Raw Donkey milk obtained from an organic breeding was serially diluted in sterile physiological solution (NaCl 0.85%) and inoculated onto MRS (Man, de Rogosa and Sharpe) agar plates specific for the isolation of Lactobacillus genus. Plates were incubated in anaerobic conditions (Anaerogen, Oxoid) for 48 h at different temperatures. Lactic acid bacteria isolates (about 150) were randomly selected from MRS-agar plates of the highest dilutions. The isolates were subcultured in MRS-broth and streaked onto MRS-agar.

Probioticity tests

1) Resistance to gastric juices

After growth, each colony was centrifuged, washed in sterile physiological solution, and re-suspended to the original volume with the same solution. The evaluation of gastric juice resistance was assayed according to De Giulio et al. « Use of alginate and cryo-protective sugars to improve the viability of lactic acid bacteria after freezing and freeze-drying » World Journal of Microbiology & Biotechnology 21 :739-746(2005), with some modifications. Aliquots were incubated in a simulated of gastric juice (pepsin 3 g/L, pH 2.5), withdrawn at different time of incubation, until 180 min, and plated. As a negative control, untreated cells were used and inoculated. The resistance to gastric juice was evaluated by the Colony Forming Units (CFU)/mL and compared with the negative control.

2) Resistance to gastric and bile salts

The colonies that were resistant to the gastric juices were incubated for a maximum of 3 h in a simulated of bile juice, composed of MRS containing bile salts 0.3%, then they were inoculated onto MRS agar plates, according to De Giulio et al. (2005). The untreated colonies were used as a negative control. The resistance to bile salts was evaluated by the Colony Forming Units (CFU)/mL and compared with the negative control.

RESULTS

Among 150 bacterial colonies isolated from raw Donkey milk, only 8 were identified as belonging Lactobacillus genus (named provisionally as Clone 32, Clone 34, Clone 37, Clone 38, Clone 41, Clone 43, Clone 48, and Clone 57), exhibited resistance to gastric juice and bile salts and showed about 75% of CFU/mL formed in comparison to the untreated control Lactobacillus put as 100%. Their optimum growth temperature was about 30 to 31°C.

Only 8 of the 150 colonies exhibited a good resistance to the simulated of gastric juice and to bile salts presence. These colonies were then studied for the following activities : hydrophobicity test, screening of cultures for bile salts hydrolyzing activity, antimicrobial activity and antibiotic sensitivity.

3) Hydrophobicity test

The capability of colonies to potentially adhere to the intestinal epithelium was evaluated by using the indirect method of hydrophobicity of Strus et al. « The in vitro activity of vaginal Lactobacillus with probiotic properties against Candida » Infectious Diseases in obstetrics and Gynaecology, 13(2) :69-75 (2005) and Ljungh et al. « High surface hydrophobicity of autoaggregating Staphylococcus aureus strains isolated from human infections studied with the salt aggregation test » Infection and immunity, 47 : 522-526 (1985). Colonies were grown in MRS broth for 24h. Microbial suspensions were mixed with equal volumes of sulphate ammonium, previously prepared with different molarities (ranging from 20 mM to 4 M). The smallest concentration of sulphate ammonium capable to cause the microbial aggregation visible onto a microscopy glass was inversely related to the salt aggregation test. An isotonic solution was used as a control.

RESULTS
- visible aggregation with 0.1 M ammonium sulphate: clones 32, 37,43 and 57
- visible aggregation with 0.5 M ammonium sulphate: clones 34,38,41 and 48.

From these data, a stronger in vitro adhesion capability to intestinal epithelium may be hypothesised for clones 32, 37, 43 and 57.

4) Screening of cultures for bile salts hydrolyzing activity

Bile salt hydrolysis is an important metabolic reaction in the bile salt metabolism of mammals. In recent years interest has increased to use bile salt hydrolysis to influence the cholesterol metabolism of humans and animals. The hydrolyzing of bile salts and the incorporation of cholesterol into the cellular membrane have the potential to lower serum cholesterol concentrations in humans. The release of free bile salts through the hydrolyzing of conjugated bile salts in the small intestine results in the excretion of more bile salts in the faeces. The primary means by which cholesterol is removed from the body is by excretion in the form of hydrolyzed bile salts. Most conjugated bile salts are re-circulated through the enter hepatic circulation. The bile salts that are excreted must be replaced by new bile acids, which are formed from cholesterol in the body. Thus, the more bile salts that are excreted, the more cholesterol is utilized from the pool within the body. Furthermore, free bile salts do not support the absorption of cholesterol and other lipids from the small intestine as well as do conjugated bile salts.

The bile salts hydrolyzing activity of the colonies was qualitatively evaluated following the method described by Minelli et al. « Assessment of novel probiotic Lactobacillus casei strains for the production of functional dairy foods » Int. Dairy J., 14 : 723-726 (2004). Briefly, overnight liquid cultures of strains (5 µL) were spotted onto MRS agar containing 0.5% conjugated bile salt mixture and 0.37 g/L of CaCl₂, and incubated as above described. The presence of the precipitated bile acid around spots (opaque halo) was considered a positive result, showing the capability of strains to hydrolyze bile salts.

RESULTS

The capability to hydrolyze bile extracts were qualitatively evaluated. All strains were capable to hydrolyze bile extracts.

5) Antimicrobial activity

The inhibition halos test on agar plate assay was employed to investigate the antimicrobial activity of the microbial strains. Samples were tested against the following bacteria :
- non-pathogenic strains : Lactobacillus acidophilus DSM 20079, Lactobacillus casei ATCC 9595, Lactobacillus bulgaricus DSM 20081, Lactobacillus sakei DSM 20494, Lactobacillus rhamnosus DSM 20711 ;
- pathogenic Gram positive strains : Bacillus cereus GN 101, DSM 4313 and DSM 4384, and Enterococcus faecalis ATCC 29212,
- pathogenic Gram negative strains : Escherichia coli (DSM 8579) and Pseudomonas aeruginosa (DSM 50071).

All strains were purchased from Deutsche Sammlung von Mikroorganismen und Zellkulturen GmbH (DSMZ Germany). Each strain was incubated at 37°C for 18 h into its specific growth medium: lactic acid bacteria were grown in Man de Rogosa Sharpe (MRS) broth (Oxoid), E. coli, E. faecalis, P. aeruginosa and B. cereus in Nutrient Broth (Oxoid). The microbial suspensions (1 x 10⁸CFU/mL) were uniformly spread onto the specific solid media plates. 50 µL of each culture were individually placed on the inoculated plates. After 30 min standing under sterile conditions at room temperature, plates were incubated at 37°C for 24 to 48 h, depending on the strain. The diameter of the clear zone shown on plates was accurately measured and it is the antimicrobial activity expressed in cm. Sterile deionised water was used as a negative control; the standard antibacterial agent, chloramphenicol, was used as a positive as in Dall'Agnol et al. « Antimicrobial activity of some Hypericum species » Phytomedicine 10: 5 1 1-5 16. (2003).

RESULTS

a) Antimicrobial activity against other Lactobacillus spp. The clones did not exhibit antimicrobial activity against Lactobacillus sakei 20494 (except the clone 57 at a very low degree).
- Clones 37, 38 and 48 exhibited a low inhibitory activity against Lactobacillus casei ATCC 9595, L. bulgaricus ATCC 11842, L. fermentum DSM 20052, L. rhamnosus DSM 2071 1
- Clone 41 exhibited a low inhibitory activity against L. fermentum DSM 20052, L. rharnnosus DSM 2071 1, L. acidophilus DSM 20079
- Clone 43 exhibited a low inhibitory activity against L. fermentum DSM 20052 and L. acidophilus DSM 20079

This result can be considered usual, due to the production by all lactic acid bacteria of antimicrobial substances, like bacteriocins, which can act not only against pathogenic bacteria, but, as "territorial defence mechanism" also against other Lactobacilli.

b) Antimicrobial activity against pathogen bacteria.

The results are shown in the following table:

| | 32 | 34 | 37 | 38 | 41 | 43 | 48 | 57 |
|---|---|---|---|---|---|---|---|---|
| B cereus GN101 | ND | ND | OX | OX | OX | ND | OX | ND |
| B cereus DSM 4313 | X | X | X | X | ND | ND | X | X |
| B cereus DSM 4384 | ND | ND | OX | OX | ND | OX | OX | ND |
| Ent. Faecalis ATCC 29212 | ND | ND | XX | XX | XX | XX | XX | ND |
| E coli HB101 | ND | X | X | X | ND | ND | X | ND |
| E coli DSM 8579 | ND | ND | X | X | ND | ND | X | OX |
| Ps aeruginosa DSM 50071 | ND | ND | XXX | X | XX | ND | X | ND |

legend:
- ND: not detectable
- OX: low inhibitory activity (halo: < 5mm)
- X: medium inhibitory activity (halo: 6-8 mm)
- XX: discrete inhibitory activity (halo from 8 to 10 mm)
- XXX: strong inhibitory activity (halo > 10 mm)

It is interesting to observe the strong antimicrobial activity exhibited by clone 37 against Ps. Aeruginosa. Clones 37, 38 and 48 exhibited a certain antimicrobial activity against the toxinogenic strain E coli DSM 8579.

6) Antibiotic sensitivity

The sensitivity of colonies to different antibiotics was evaluated by using 30-40 mg of the following antibiotics:
- ampicillin, and amoxicillin as inhibitor of cell wall synthesis;
- gentamicin sulfate, lincomycin, streptomycin sulfate, tetracycline, chloramphenicol and spiramycin as inhibitors of protein synthesis;
- rifamixin as inhibitors of nucleic acid synthesis.

Each of the antibiotic powders was carefully weighed, dissolved, diluted in appropriate diluents and filter sterilized prior to addition to MRS medium. Plates were inoculated with LAB strains, and incubated as above described. The sensitivity or resistance to antibiotics was evaluated by the inhibition halo test.

RESULTS

The clones exhibited a variable degree of sensitivity to the antibiotics used in the test. As shown in the table below, clone 32 was sensitive to all antibiotics; on the other hand, the other clones were more or less resistant against streptomycin and lyncomycin.

| Clone | Antibiotic sensitivity |
|---|---|
| 32 | sensitivity to all antibiotics tested |
| 34 | sensitivity to all antibiotics tested |
| | resistance against streptomycin |
| | low resistance against lincomycin |
| 37 | sensitivity to all antibiotics tested |
| | low resistance against streptomycin and lincomycin |
| 38 | sensitivity to all antibiotics tested |
| | resistance against streptomycin and lincomycin |
| 41 | sensitivity to all antibiotics tested |
| | resistance against streptomycin |
| | low resistance against lincomycin |
| 43 | sensitivity to all antibiotics tested |
| | resistance against streptomycin |
| | low resistance against lincomycin |
| 48 | sensitivity to all antibiotics tested |
| | resistance against streptomycin |
| | low resistance against lincomycin |
| 57 | sensitivity to all antibiotics tested |
| | resistance against streptomycin |
| | low resistance against rifamixin |

Phenotypic and genotypic approaches (Metabolism of sugars, DNA fingerprint, DNA-DNA Hybridation, 16 S RNA sequence comparison)

7) Metabolism of sugars

Isolates were identified to the species level using the carbohydrate fermentation API 50 CHL (BioMérieux) identification system. API 50 CHL Medium, intended for the identification of the genus Lactobacillus and related organisms, is a ready-to use medium which enables the fermentation of 49 carbohydrates on the API 50 CH strip to be studied. A suspension is made in the medium with the microorganism to be tested and each tube of the strip is inoculated. During incubation, carbohydrates are fermented to acids which produce a decrease in the pH, detected by the colour change of the indicator. The results make up the biochemical profile of the strain and are used in its identification or typing. The presumed probiotic strains were inoculated in API 50 CHL strips and evaluation of colour changes was performed after 24 and 48 h of incubation at 37°C.

Each strip was composed as shown in the table below. The results were analysed by using an API BioMérieux software.

| **STRIP 0-9** | **STRIP 10-19** | **STRIP 20-29** |
|---|---|---|
| **tube / substrate** | **tube / substrate** | **tube / substrate** |
| 0 CONTROL | 10 GALactose | 20 α-Methyl-D-Mannoside |
| 1 GLYcerol | 11 GLUcose | 21 α-Methyl-D-Glucoside |
| 2 ERYthrytol | 12 FRUctose | 22 N-Acetyl-Glucosamine |
| 3 D ARAbinose | 13 MaNnosE | 23 AMYgdalin |
| 4 L ARAbinose | 14 SorBosE | 24 ARButin |
| 5 RIBose | 15 RHAmnose | 25 ESCulin |
| 6 D XYLose | 16 DULcitol | 26 SALicin |
| 7 L XYLose | 17 INOsitol | 27 CELlobiose |
| 8 ADOnitol | 18 MANnitol | 28 MALtose |
| 9 β-Methyl-D- | 19 SORbitol | 29 LACtose |
| Xyloside | | |

| **STRIP 30-39** | **STRIP 40-49** | |
|---|---|---|
| **tube / substrate** | **tube / substrate** | |
| 30 MELibiose | 40 D TURanose | |
| 31 Sucrose | 41 D LYXose | |
| 32 TREhalose | 42 D TAGatose | |
| 33 INUlin | 43 D FUCose | |
| 34 MeLeZitose | 44 L FUCose | |
| 35 RAFfinose | 45 D ARabitoL | |
| 36 Starch | 46 L ARabitoL | |
| 37 GLYcoGen | 47 GlucoNaTe | |
| 38 XyLiTol | 48 2-Keto-Gluconate | |
| 39 GENtobiose | 49 5-Keto-Gluconate | |

### RESULTS of fermentation tests (API)

Clone 32: insufficient discrimination
Clone 34 : insufficient discrimination
Clone 37: excellent identification with Lactobacillus plantarum 99.5%
Clone 38: doubt identification
Clone 41: preliminary doubt identification
Clone 43: preliminary doubt identification
Clone 48: excellent identification with Lactobacillus plantarum 99.5%
Clone 57: insufficient discrimination

Genetic approach

The following methodologies were applied for the molecular identification: DNA fingerprint, DNA-DNA Hybridization and 16 S RNA sequencing.

8) DNA fingerprinting

Random Amplified Polymorphic DNA-PCR (RAPD-PCR) assay was used to produce fingerprint patterns according to Ronimus et al. (1997). DNA amplification was performed in a 50 µL PCR reaction mixture containing: 50-200 ng of genomic DNA, 1X PCR buffer (supplied as component of the DNA polymerase kit), 3 mM MgCl₂, 250 µM dNTPs, 0.5 µM of OPR-2 primer (5'-CACAGCTGCC-3', sequence SEQ ID NO: 1) or OPR-13 primer (5'-GGACGACAAG- 3', sequence SEQ ID NO: 2) and 2.5 units of Platinum® Taq DNA polymerase (INVITROGEN). The mixtures were amplified in a thermocycler iCycler® (BIO RAD). The amplification profile consisted of an initial denaturation of 2 min at 92°C and 35 cycles of 15 sec at 94°C, annealing for 15 sec at 36°C (previously optimized by temperature gradient amplification) and elongation for 2 min at 72°C. A final extension of 7 min was carried out at 72°C. 10-20 µL of PCR products were electrophoresed on DNA 7500 microchip (Agilent) using a 2100 Bioanalyzer equipped by a 2100 EXPERT software (Agilent).

RESULTS

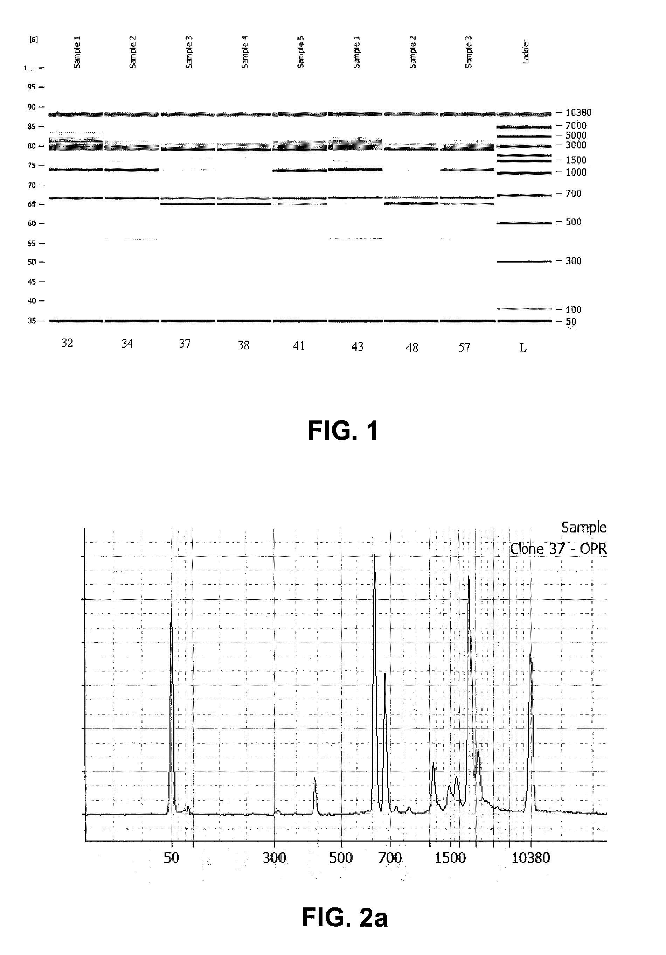

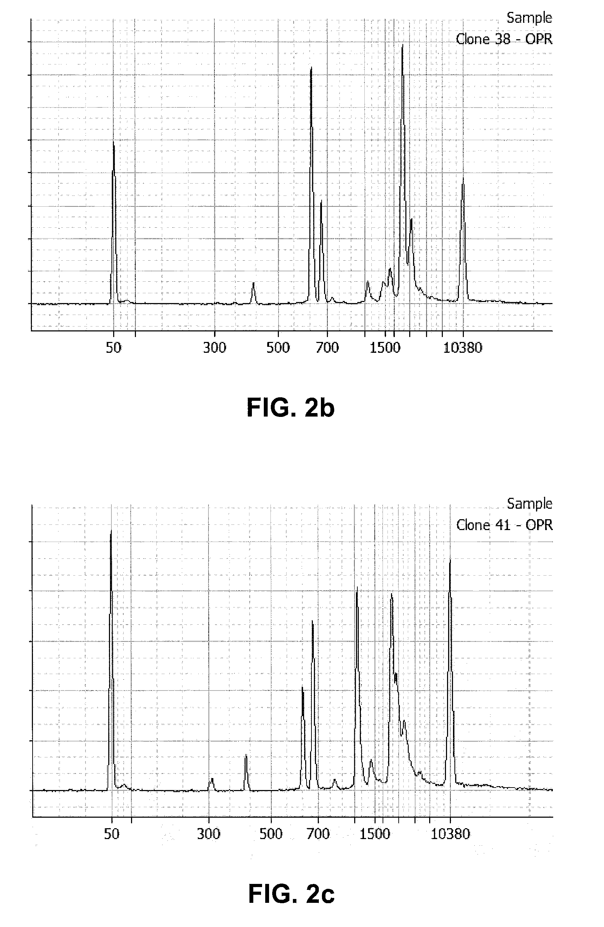

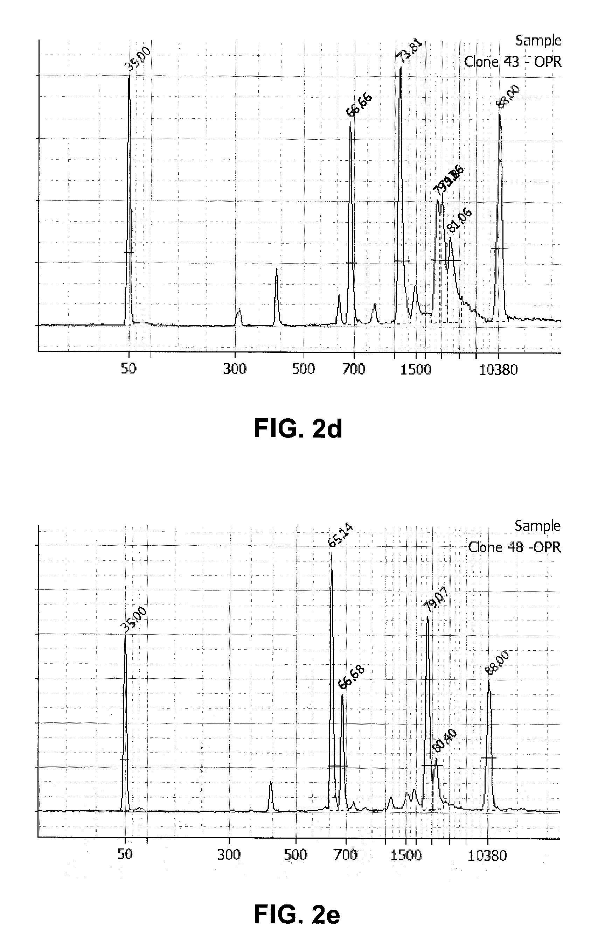

The API tests were followed by genotypic studies. DNA fingerprinting analysis (Figure 1) shows that clones 37, 38 and 48 seem to belong to the same species. In Figures 2a to 2e, the electropherograms relative to clones 37, 38, 41, 43 and 48 are provided.

9) 16 S RNA sequence comparison

Total RNA was extracted by RiboPure-Bacteria kit (Ambion) following manufacturer's instructions. RNA was dissolved in RNA-storage-solution (Ambion), UV-quantified by a Bio-Photometer® (Eppendorf) and stored to - 80°C. RNA aliquots (6 µg) were digested by RNAse-free DNAse I (Ambion DNA-freeTMk it) in a 20 µL final volume reaction mixture, to remove contaminating genomic DNA. After DNAse digestion, concentration and purity of RNA samples were evaluated by the RNA-6000-Nano® microchip assay, using a 2100 Bioanalyzer® equipped with a 2100-Expert-Software® (Agilent), following the manufacturer' s instructions. For all samples tested the RNA integrity number (R.I.N.) was greater than 7 (relatively to a 0-10 scale). were reverse transcribed in a 20 µL reaction mixture. A mixture containing 3 µg of total RNA, as evaluated by the 2100 Bioanalyzer, 2 pmoles of 1517R forward primer (see before), 2 mM dNTPs in a total volume of 10 µL was incubated for 2 min at 70°C and quickly cooled to 40°C. To the mixture were added 10 µL containing 2x of a suitable buffer (Invitrogen) 20 mM dithiothreitol, 20 units of RNAse inhibitor (Invitrogen), and 200 units of MoMuLV Superscript@ III reverse transcriptase (Invitrogen). The reaction mixture (final volume 20 µL) was incubated at 55°C for 60 min and at 70°C for 15 min to stop the reaction and for RNA degradation. Amplification of 16S cDNA was performed by an iCycler-iQ5® in a 50 µL reaction mixture containing: 1x of a suitable buffer (Invitrogen), 200 pM dNTPS, 300 nM of bacterial 16S general primers designed on E. coli 16S RNA sequence accession (8-Forward and 1517-Reverse). The amplification profile consisted of an initial denaturation of 3 min at 94°C, 25 cycles of 30 sec. at 94°C, annealing for 30 sec. 57°C and elongation for 1 min at 72°C and final elongation of 7 min at 72°C. 5 µL of PCR products were electrophoresed on 1% agarose gel (Agarose -1000, Invitrogen) in 1x TAE buffer at 5 V/cm for 4h. Ethidium bromide (0.1 µg/mL) was included both in the gel and electrophoresis buffer and PCR products were detected by UV visualization and recorded on Polapan 55 films (Polaroid). 100-150 µL of each amplification mixture were air dried and send to MWG (Ebersberg - Germany), for sequencing. Sequencing was performed using the primers 8F, 1517 R and by the internal primer 368 F, designed on the basis of the first sequencing data. Sequence-conting was perforrned by the DNA-Baser vers. 2.0 software utilizing the ".scf" sequence files. Generated ".fasta" sequences (about 1400 bases long) were aligned at NBCI -BLAST and at "Ribosome Data Base Project" -RDP data banks.

RESULTS

Ribosomal RNA was extracted and retro-transcribed. cDNA obtained was amplified and sequenced as described above. Sequence-contings of about 1400 bases were obtained and compared in data banks. Below is a synthesis of sequence comparisons at Ribosomal Database Project (RDP). A picture is emerging that includes our clones into the very tight cluster of L. plantarum, paraplantarum and pentosus species that present (type strains) a 16 S RNA identity greater than 99.9%. The results given below are percentages of 16 S RNA identity of the clones of the invention compared to these species.
Clone 41
domain Bacteria (20) (match sequences)
phylum Firmicutes (20) class "Bacilli" (20) order "Lactobacillales" (20) family Lactobacillaceae (20) genus Lactobacillus (20)
0.994 1400 L. paraplantarum (T); DSM 10667T; A5306297
0.979 1405 L. plantarum (T); JCM 1 149; D792 10
0.984 1410 L. pentosus (T); JCM 1558; D79211
Clone 43
domain Bacteria (20) (match sequences)
phylum Finnicutes (20) class "Bacilli" (20) order "Lactobacillales" (20) family Lactobacillaceae (20) genus Lactobacillus (20)
1400 L. paraplantarum (T); DSM 10667T; AJ306297
0.985 1405 L. plantarum (T); JCM 1149; D79210
0.990 1410 L. pentosus (T); JCM 1558; D79211
Clone 48
domain Bacteria (20) (match sequences)
phylum Firmicutes (20) class "Bacilli" (20) order "Lactobacillales" (20) family Lactobacillaceae (20) genus Lactobacillus (20)
1.000 L. plantarum (T); JCM 1 149; D792 10
Clone 57
domain Bacteria (0/20/5164) (selected/match/total RDP sequences)
phylum Firmicutes (0/20/1178) class "Bacilli" (0/20/697) order "Lactobacillales" (0/20/289) family Lactobacillaceae (0/20/114) genus Lactobacillus (0/20/104)
0.987 1400 L. paraplantarum (T); DSM 10667T; A5306297
0.990 1405 L. plantarum (T); JCM 1149; D79210
0.994 1410 L. pentosus (T); JCM 1558; D79211

10) DNA-DNA Hybridization

For Quantitative DNA-DNA hybridisation and homology percentage calculation the DNA was extracted and purified from bacterial cell culture (about 250 mg of dry pellet for each strain) using the Genomic-DNA-Buffer Set and the Genomic-tip-100/G columns (QIAGEN SPA, Milano Italy), according to manufacturer's instructions with minor modifications. DNA was dissolved in TE buffer (10 mM Tris pH 8, 1 mm EDTA) and serial diluted to a final concentration (WS) of 50 µg/mL, as evaluated by UV-absorbance using a Bio-Photometer (Eppendorf, Germany). WS DNA concentration was confirmed by fluorimetric measurements using the Quant-iT DNA assay Kit (INVTTROGEN, Milano Italy); DNA size was estimated by 0.8% DNA-grade agarose (BIO-RAD) electrophoresis using DNA as molecular weight marker (DNAs size about 32 kD). WS solutions were diluted to a final concentration of 2 ng/mL in 0.1 X SSC containing 5 ng/mL herring sperm DNA. DNA was denatured by 10 min at 100°C and quick immersion in water-ice bath. 50-100 ng of DNA for each strain were blotted in quadruplicate on nylon membrane positively charged (Roche, Germany) by using a Dot-Blot apparatus (Bio-Rad, Ca USA) connected to a soft vacuum. A standard curve 20 to 200 ng DNA/spot of homologous DNA (the DNA to probe) was included in the dot. The DNA was cross-linked to nylon by 3 min UV exposure and by 1 hr backing under-vacuum at 120°C. Membranes were frozen at -20°C until analysis.

1 µg of DNA-to-probe was labelled overnight with digoxigenin-dUTP in a 20 µL reaction mixture using the Random Primed DNA labelling kit (Roche, Germany) according to manufacturer's instructions. Membranes were pre-hybridizated for 3 hrs at 40°C in DIG Easy-Hyb solution (Roche) and hybridizated over-night at 40°C in DIG Easy-Hyb solution containing 20 pg/mL of Dig-labelled probe (heat-denatured as described above), using a tube-rotating hybridization incubator (GFL). Stringency washes were : twice for 5 min at room temperature in 2 x SSC solution containing 0.1 SDS, twice for 15 min at 68°C in 0.1 x SSC solution containing 0.1 x SDS. Immune-detection was performed using the anti-Digoxigenin-AP antibody (anti-digoxigenin FAB fragment conjugated to alkaline-phosphatase), the CDP-Star chemiluminescent substrate and the DIG Wash and Block buffer set Kit, all reagents and relative instructions were from Roche. Chemiluminescence was quantified in condition of time-exposure linearity by using a VersaDOC 4000 apparatus (BIO-RAD) equipped by the Quantity-one software. The DNA-DNA homology percentage was calculated according to Jahnke (1994) by putting as 100 % the media of the chemiluminescence values (Adjusted Volume Intensity x mm² ) acquired from the homologous DNA spots and taking in account the linear response of the homologous DNA standard curve. The media standard deviation of replicate samples did not exceeded 5% of the media value.

RESULTS

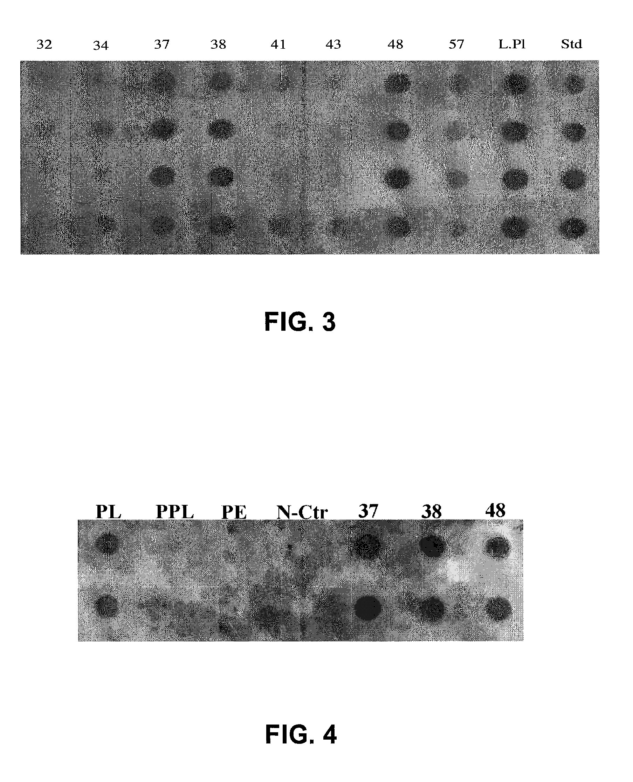

Metabolic data were confirmed by DNA-DNA hybridization, utilizing L. plantarum DNA labelled by dUTP-dioxigenin as a probe. As shown by Figure 3, the clones 37, 38, 48 showed 86, 99, 99 % respectively of DNA-DNA homology with L. plantarum (L. PI on Figure 3), knowing that the taxonomic DNA-DNA limit for different species is below 70% of DNA-DNA homology.

To distinguish from the three possibilities and to confirm previous findings, clones 37, 38 and 48 were DNA-DNA hybridised within L. plantarum (PL on figure 4), L. paraplantarum (PPL on figure 4) and L. pentosus (PE on figure 4) strain-type DNAs, to a probe of labelled DNA from clone 37 (Fig 4). N-Ctr is negative control.

The foregoing description of preferred embodiments of the invention is not intended to be exhaustive or to limit the invention to the disclosed embodiments. Various changes within the scope of the invention will become apparent to those skilled in the art and may be acquired from practice of the invention.

Based on the experimental results of DNA-DNA hybridization and the analysis of 16S RNA, the strains 37, 38 and 48 belong to a unique cluster, in which L. plantarum, L. pentosus and L. paraplantarum are also present, but are different from them. It is considered that strains 37, 38 and 48 represent a subspecies of L. plantarum, which will be named L. plantarum asini. For similar reasons, the strains 41 and 43 apparently belong to a new species, Lactobacillus asini. Indeed, strains 41 and 43 present a 40-50% of similarity with the other lactobacilli, mainly with pentosus, plantarum and paraplantarum.

Strains 37, 38 and 48, and strains 41 and 43, are microorganisms belonging to genus Lactobacillus, with a homology of the 16S RNA higher than 99.2% with the strict cluster of L plantarum , L paraplantarum and L pentosus, and with relative finger print and protein profile proportional to such homology.

SEQUENCE LISTING

SEQ ID NO: 1
LENGTH: 10 bases
TYPE: nucleic acid
STRANDENESS: single
TOPOLOGY: linear
MOLECULAR TYPE: DNA
SEQUENCE:
CACAGCTGCC

SEQ ID NO: 2
LENGTH: 10 bases
TYPE: nucleic acid
STRANDENESS: single
TOPOLOGY: linear
MOLECULAR TYPE : DNA
SEQUENCE:
GGACGACAAG

SEQUENCE LISTING

SEQ ID NO: 1
<210> 1
<211> 10
<212> DNA
<213> OPR-2 primer

<400> 1
cacagctgcc 10

SEQ ID NO: 2

<210> 2
<211> 10
<212> DNA
<213> OPR-13 primer

<400> 2
ggacgacaag 10

## Claims

1. Probiotic lactic bacteria isolated from Donkey milk.

2. Bacteria according to claim 1 selected among the species Lactobacillus plantarum, Lactobacillus paraplantarum, Lactobacillus pentosus, Lactobacillus asini.

3. Bacteria according to claim 2 selected among the subspecies Lactobacillus plantarum asini.

4. Bacteria according to any one of claims 1 to 3, selected from the group consisting of strains deposited under accession number DSM 22098, DSM 22099, DSM 22100, DSM 22102, and DSM 22101 with DSMZ.

5. Composition comprising one or a plurality of the probiotic lactic bacteria species or strains according to any one of claims 1 to 4.

6. Composition according to claim 5, wherein the composition is a food composition or a beverage composition.

7. Process for manufacturing a food composition or a beverage composition containing at least the steps of:
- inoculating a food product with viable probiotic lactic bacteria according to any one of claims 1 to 4
- placing the inoculated food product under conditions favorable to the metabolism of said probiotic lactic bacteria
- fermenting said food product until a population of at least [10⁶] CFU / mL of food product is reached.

8. Process for manufacturing a food composition or a beverage composition containing at least the steps of:
- fermenting probiotic lactic bacteria according to any one of claims 1 to 4 until a population of at least [10⁶] CFU / mL is reached,
- protecting said probiotic lactic bacteria,
- admixing a food product with said protected probiotic lactic bacteria.

9. A process according to claim 7 or 8, wherein fermentation is stopped when it has reached the stationary phase of fermentation, preferably within about 24 hours of having placed said probiotic lactic bacteria under conditions favorable to their metabolism.

10. Use of probiotic lactic bacteria according to any one of claims 1 to 4 in the preparation of a composition for treating a disorder associated with the colonization of a mucous membrane by a pathogenic microorganism.

11. Use according to claim 10, wherein said mucous membrane is selected in the group consisting of the gut mucosa and the stomach mucosa.

12. Use according to claim 10 or 11, wherein said pathogenic microorganisms is an enteropathogens, such as Salmonella enteriditis, Vibrio cholerae, Escherichia coli.

13. Use of probiotic lactic bacteria according to any one of claims 1 to 4 for protecting fermented food products against food pathogens, such as Lysteria or Salmonella, Campylobacter or Clostridium, by inhibiting the development of such food pathogens.
